Europäisches Patentamt

European Patent Office

Office européen des brevets

(11) Publication number: **0 227 102**
A2

# (12) EUROPEAN PATENT APPLICATION

(21) Application number: 86117947.1

(22) Date of filing: 23.12.86

(51) Int. Cl.⁴: **C12N 5/00** , C12N 9/72

(30) Priority: 27.12.85 JP 292481/85

(43) Date of publication of application:
01.07.87 Bulletin 87/27

(84) Designated Contracting States:
CH DE GB LI

(71) Applicant: Wakamoto Pharmaceutical Co.,
Ltd.
No. 8, Muro-machi 1-chome Nihonbashi
Chuo-ku
Tokyo(JP)

(72) Inventor: Matsuo, Osamu
1-29-18, Ohnodai Sayama-cho
Minami-Kawauchi-gun Osaka-fu(JP)
Inventor: Fukao, Hideharu
5-5-1-601, Nishiyamadai Sayama-cho
Minami-kawauchi-gun Osaka-fu(JP)
Inventor: Yokoigawa, Kumio
857-2, Kaneko Ohi-machi
Ashigarakami-gun Kanagawa-ken(JP)
Inventor: Ohmura, Takeo
280, Kanate Ohi-machi
Ashigarakami-gun Kanagawa-ken(JP)
Inventor: Akiba, Kiyoshi
3, Kopo-sazanka 812-1, Shibusawa
Hatano-shi Kanagawa-ken(JP)

(74) Representative: Vossius & Partner
Siebertstrasse 4 P.O. Box 86 07 67
D-8000 München 86(DE)

(54) Cell line established from human normal uterine muscle, method for producing plasminogen activator using the cell line, and plasminogen activator.

(57) Described is a cell line (KW strain IFO No. 50113) which is established from human uterine muscle. The cell line can be subcultivated 120 times or more and permits the production of a plasminogen activator on a commercial scale, and the plasminogen activator with high safety can be used to treat thrombosis and improve the antithrombotic properties of various medical materials such as artificial organs, artificial blood vessels, plasma exchange system, catheter, etc.

## Cell Line Established from Human Normal Uterine Muscle, Method for Producing Plasminogen Activator Using The Cell Line, and Plasminogen Activator

Background of The Invention

Field of the Invention:

The present invention relates to a cell line established from human normal uterine muscle tissue, a method for producing a plasminogen activator by cultivating the cell line, and a new plasminogen activator produced by the cell line.

Description of Prior Art:

Several plasminogen activators are widely distributed in most tissues and body fluids. The plasminogen activator enzymatically converts plasminogen to plasmin, which lyses fibrin. In addition, the plasminogen activator may play important roles in other functions including ovulation and metastasis of neoplastic cells. The study of the plasminogen activator on fibrinolytic functionhas especially been developed and the plasminogen activator is applied in clinical use. For example, the plasminogen activator has been widely utilized as a medicine for treating thrombosis and as an enzyme for imparting thrombolytic characteristics to medical materials such as artificial organs, artificial blood vessels, plasma exchange systems, catheters and the like.

Up to now, as plasminogen activators, there have been known urokinase (hereinafter referred to as UK) derived from urine, and tissue plasminogen activators (hereinafter refered to as TPA) produced by animal or human cells in culture, and extracted from human tissue. However, there are a lot of problems relating to their quality and commercial production.

UK is the first plasminogen activator that is put into practical use and has been widely used as a medicine at present. However, it has been found out that UK has a drawback of causing bleeding easily, because UK has a low affinity to fibrin and activates the fibrinogenolytic system of the whole blood, resulting in the degradation of fibrinogen. Subsequently, TPA produced from human melanoma cells or some other tumor cell lines was found as a fibrinolytic enzyme capable of overcoming the above problems provided by UK. TPA differs from UK in its immunochemical property. And, TPA has a strong affinity for fibrin since it has fibrin binding sites and specifically lyses only thrombus, and therefore it is expected to be an excellent thrombolytic agent free from any side effects.

However, since the melanoma cell is a kind of tumor cell, there is a doubt about its safety when it is used as a material for producing medical products. That is, the product from such tumor cells in culture has the problem concerning antigenicity and carcinogenicity. For that reason, many attempts to obtain cells for producing such medical products other than the melanoma cell have been conducted, and as a result there have been proposed various TPA's derived from various kinds of tissues and cells such as those explained below:

(A) TPA produced by the melanoma cell (Japanese Patent Disclosure Nos. 57-28009 and 59-4232l; Journal of Biological Chemistry 1981, 256, 7035-704l);

(B) TPA from human uterine extract (Biochimica et Biophysica Acta, 1979, 580, 140-153);

(C) TPA from human vessel wall extract (Journal of Biological Chemistry, 1979, 254, 1998; Japanese Patent Disclosure No. 59-l3732);

(D) TPA from human kidney extract (Biochimica et Biophysica Acta, 1982, 7l7, 327; Japanese Patent Disclosure No. 59-l3732);

(E) TPA produced from cells derived from human normal tissue (Japanese Patent Disclosure Nos. 59-5l220 and 60-l58ll7);

(F) TPA produced from normal diploid fibroblast derived from human fetal lung (Abstract of Papers, the Meeting held by Nippon Nogei Kagaku Kai, l985, p 330); and

(G) TPA produced from the mutant KYM-A of human rhabdomyoma cell (Japanese Patent Disclosure No. 60-l58ll5/l985).

All of the TPA's mentioned above have a higher affinity for fibrin than UK, so that they are expected to be medicines for thrombosis.

However, since the Products from tumor cells in culture have the problem with antigenicity and carcinogenicity, it is necessary that the cell lines derived from tumor cells should be changed to other new ones using the gene recombinant technique. Moreover, in this case, human normal cells must be used if a host cell is selected so that the sugar and the conformation of protein as well as the primary structure of protein (amino acid sequence) of the TPA, are identical with those of the natural one.

On the other hand, it is practically quite difficult to industrially extract a great deal of TPA from human normal tissues. On the other hand, in cell culture, the human normal cells have big restriction on their degree of proliferation, and in most cases they die until 50 generations of cell divisions, and therefore, if one intends to industrially produce TPA, it is required to continuously collect new tissues therefor and select cells therefrom satisfying desired requirements, which are quite difficult and troublesome operations.


Summary of the Invention

Thus, the principal object of this invention is to provide a cell line derived from human normal tissue and capable of producing TPA on an industrial scale.

Another object of this invention is to provide a cell line having high safety and capable of bearing almost permanent subcultivation.

A further object of this invention is to provide a method for producing TPA on an industrial scale.

A still another object of this invention is to provide a new TPA having an excellent quality such as high affinity for fibrin and being free of any side effects.

In order to achieve the above objects, the present inventors have selected human normal uterine muscle tissue which is expected to be able to produce TPA and have made intensive study so as to obtain a spontaneous transformant strain capable of permanent subcultivation, by repeating the subcultivation from the primary culture of the tissue. As a result, the inventorshave successfully obtained an established cell strain which satisfys the above objects and have established a process which is advantageous to industrially manufacture TPA by the cultivation of the cell strain.

Incidentally, the inventors named a new established cell line which the present inventors had succeeded to obtain as "KW strain established from human normal uterine muscle" and filed an application for depositing the cell line to Fermentation Research Institute (FRI). However, such an application was rejected for the reason that such a cell line is beyond the FRI regulations. Thereafter, on the other hand, the inventors deposited the cell line to Institute for Fermentation, Osaka (IFO) as IFO No. 50ll3.

Firstly, the present invention relates to a cell line (KW strain) which is established from human normal uterine muscle and has the following physical and chemical characteristics:

l) Producing a plasminogen activator which is immunologically different from urokinase;

2) Having estrogen receptors specific to the uterine cells;

3) Being capable of subcultivation of l20 or more; and

4) Having doubling time of l.4 to l.6 days.

Secondly, the present invention relates to a process for producing a plasminogen activator comprising cultivating the above KW strain.

Finally, the present invention relates to a plasminogen activator having the following physical and chemical properties:

(l) Specific activity:
over 90,000 IU/mg-protein

(2) Molecular weight:
70,000 ±2,000 (SDS-PAGE)
67,000 ±5,000 (Gel filtration)
67,000 (HPLC)

(3) Molecular structure:
Single stranded structure

(4) N-terminal amino acid sequence:

```
  1                 5                    10  11
Ser-Tyr-Gln-Val-Ile-*-Arg-Asp-Glu-Lys-Thr-Gln-Met-Ile-
  15                       20  21                    25
Tyr-Gln-Gln-His-Gln-**-Trp-Leu-Arg-Pro-Val-Leu-
                  30  31                35
Arg-**-Asn-Arg-Val-Glu-Tyr-*-Trp-*-Asn-
```

wherein the amino acids indicated by * and ** are not confirmed. However, the former is assumed to be Cys and the latter Ser.

(5) Substrate specificity:

The plasminogen activator hydrolyzes the synthetic substrate S-2251 in the presence of plasminogen, and more specifically hydrolyzes S-2288 than S-2444.

(6) Optimum pH: l0

(7) pH Stability:

The plasminogen activator is stable under a weak alkaline condition (pH: 8.0 -9.0).

(8) Thermal stability:

The plasminogen activator is stable at 45°C for l0 hours (at pH 9.0) and loses almost l00% activity within 30 minutes at 90°C and pH 9.0.

(9) Reaction velocity constant at 37°C and pH 8.4:

| Synthetic Substrate | Km (mol/ℓ) | Kcat (sec⁻¹) |
|---|---|---|
| S-2288 | $4.3 \times 10^{-4}$ | 3.5 |
| S-2444 | $10.0 \times 10^{-4}$ | 1.9 |

Note: Km is the Michaelis constant and Kcat is the catalytic rate constant.

(l0) Isoelectric point: 4.5 - 7.2

As measured by the chromatofocusing method using Mono P column (Pharmacia).

The present invention will be described in detail below.

Firstly, the experiment to obtain the present KW strain will be explained below. However, this experiment is to merely explain the progress wherein the present invention has been achieved, but is not to show that the present KW strain is reproducibly obtained by this experiment.

Detailed Explanation of The Invention

(Experiment for Obtaining KW Strain)

Human uterine normal muscle layer was cut off from the human uterine obtained at hysterectomy, minced to pieces having a size of l to 2 mm³ with scissors after washing with Eagle's minimum essential medium (hereunder referred to as MEM), and then centrifuged at 800 to l,000 rpm for 5 minutes after washing with MEM, so as to collect tissues.

To the resultant tissues, there was added about fivefold enzyme solution (200 U/ml of collagenase and 0.05 to 0.l% of trypsin) and the mixture was stirred at 37°C. While monitoring the degree of digestion, the nondigested tissues were removed by a nylon mesh after the digestion for 2 to 4 hours and then the filtrate was subjected to centrifugation, to collect cells. After washing the cells with MEM several times, they were seeded in a plastic dish having a diameter of 60 mm. MEM containing a l0% fetal calf (bovine) serum was added to the dish and then the cells were cultivated at 37°C in an atmosphere of 5% $CO_2$ -95% air. After the cells were sufficiently proliferated, the grown cells were treated with the above enzyme solution, and collected from the plastic dish, and then 2 × l0⁵ cells thereof were again seeded in a 25 cm² plastic laboratory dish. l0 ml of MEM containing l0% fetal calf serum was added to this dish and then the cells were cultivated at 37°C in an atmosphere of 5% $CO_2$ -95% air. When this subcultivation procedure was repeated l8 times, cells having an extremely higher proliferation rate than that of the original cells appeared and rapidly prevailed in the culture flask, which were collected and named as KW strain.

4

In general, it has been believed that the number of cell divisions of a human normal cell is at most 50 ±I0 times. However, the obtained cell have good potency even after more than I00 cell divisions, and it was found that these cells were spontaneous transformant capable of bearing almost permanent subcultivation. Moreover, it was also confirmed that the conditioned medium included TPA which immunologically differred from UK.

Incidentally, this KW strain can be stored for a long period of time in liquid nitrogen by dispersing them in MEM containing a I0% dimethyl sulfoxide and a I0% fetal calf serum as cryoprotectant, freezing the dispersion according to a slow freezing technique wherein a cooling rate is I to I.5°C per minute.

This strain is stored at Research Department of Biological Chemistry of Wakamoto Pharmaceutical Co., Ltd. It is also stored at IFO under the depository number of IFO 50II3 as stated above.

(Properties of KW strain)

I. Material producibility:

The strain produces a plasminogen activator which is immunochemically different from UK.

2. Receptor:

The strain contains estrogen receptors peculiar to the uterine cell and its number is varied depending on the cell cycle.
Growth phase: about 2.7 $\times$ I0$^6$/cell
Stationary phase: about I.0 $\times$ I0$^6$/cell
Subcultivation: The strain can be subcultivated more than I20 times.
Doubling time: I.4 -I.6 day
(in Eagle's MEM containing a I0% fetal calf serum)
Incidentally, the number of estrogen receptors was determined as follows:
The cells in growth phase or stationary phase were incubated at 37°C for 2 hours in MEM containing - [$^3$H] estradiol-I7 $\beta$(0 -I00 nM). After the incubation, the incorporation of [$^3$H] estradiol-I7 $\beta$ into the cells was determined from the radioactivity of the cell lysate. The number of receptors was determined by the scatchard plot analysis (Zava, D.T. and McGuire, W.L., J. Biol. Chem., I977, _252_, 3703-3708).
KW cells may, for example, be cultivated according to any one of the following two methods:

(a) Monolayer Cultivation

.A desired number of KW cells are seeded in a culture vessel containing a suitable culture medium including serum. The cell cultivation is generally continued for 7 to I0 days at about 37°C in an atmosphere of, for example, 5% $CO_2$ and 95% air, to form the monolayer. Then, resulting cells are generally washed with, for instance, phosphate buffered saline and incubated with a suitable medium without serum at about 37°C for I0 to 20 days in atmospheric air.

Examples of the culture medium usable for the present invention include MEM, Dulbecco's modified MEM, I99 medium, Ham's medium, etc. which contain serum such as fetal calf serum (FCS), as the cell growth medium. Preferred medium for producing TPA includes those listed above without serum such as FCS.

(b) Microcarrier Cultivation

The microcarrier cultivation may be carried out, for instance, in a spinner flask containing Cytodex I, KW cells and a suitable medium at about 37°C in atmospheric air for about 40 days at about 80 rpm. An about 80% medium replacement may be employed every day.

TPA produced may be separated from the conditioned medium and purified according to any of the conventional methods for separating and purifying proteins, such as chromatographic techniques, for example, adsorption chromatography, ion-exchange chromatography, gel filtration, different kinds of affinity chromatography, fractional precipitation, membraneseparation, electrophoresis, high pressure liquid chromatography (HPLC), etc.

The fibrinolytic activity of TPA is determined on bovine fibrin plates (see, Matsuo, O. et al., Acta Haematol. JAP., 1976, 39, 298-305) and with the synthetic chromogenic substrates S-2288, S-2251 and S-2444 (Kabi Diagmostica; H-D-Ile-Pro-Arg-pNA, H-D-Val-Leu-Lys-pNA and Glu-Gly-Arg-pNA, respectively) and expressed as IU (International Unit) with the standard urokinase.

The molecular weight of the TPA is determined by SDS-polyacrylamide gel electrophoresis (SDS-PAGE), gel filtration and electrophoretic enzymography (enzymography: Matsuo, O. et al., Hemostasis, 1986, 16, 43-50). Kinetic studies of TPA is carried out with S-2444 and S-2288. The isoelectric points is determined in Mono P column (Pharmacia) by chromatofocusing with Fast Protein Liquid Chromatography System (Pharmacia). The affinity of TPA to fibrin is examined by chromatography on fibrin-Sepharose. This fibrin-Sepharose is obtained by treating the fibrinogen-Sepharose with thrombin. The immunological study on TPA is performed with anti-UK antibody (see, Wun, T.C. et al., J. Biol. Chem., 1982, 257, 3276-3283) and anti-TPA antibody (melanoma TPA) (Rijken, D.C. et al., Hemostasis, 1982, 11 (Suppl), 31). Amino acid sequence analysis is performed by PTC method (phenyl isothiocyanate method) with a gas phase protein sequencer (Applied Biosystems, Model 470A).

The present invention will hereunder be explained more in detail with reference to the following examples.

Example I

KW cells obtained according to the above method were seeded in 20 culture vessels of 1200 cm² (1 × 10⁷ cells per culture vessel). 180 ml of MEM containing a 10% fetal calf serum and a protease inhibitor was added thereto as a growth medium, and then the cells were cultivated at 37°C for 6 days in an atmosphere of 5% $CO_2$ -95% air. After washing with physiological saline, 200 ml of 199 medium containing a protease inhibitor and free from serum was added to each of the culture vessels and the cells were cultivated to produce TPA for 18 days while the medium replacement was carried out every day.

TPA was purified from the conditioned medium according to the method of D.C. Rijken and D. Collen (J. Biol. Chem., 1981, 256, 7035-7041) wherein TPA was purified by chromatography on zinc chelate-Sepharose, chromatography on concanavalin A-Sepharose, gel filtration in this order and further was subjected to lysine-Sepharose chromatography and gel filtration, so as to obtain electrophoretically pure TPA.

TPA had the following physico-chemical properties:

(1) Specific activity:

over 90,000 IU/mg-protein

(2) Molecular weight:

70,000 ±2,000 (SDS-PAGE)

67,000 ±5,000 (Gel filtration)

67,000 (HPLC)

(3) Molecular structure:

Single stranded structure

(4) N-terminal amino acid sequence:

```
    1                 5                    10   11
    Ser-Tyr-Gln-Val-Ile-*-Arg-Asp-Glu-Lys-Thr-Gln-Met-Ile-
    15                  20   21                  25
    Tyr-Gln-Gln-His-Gln-**-Trp-Leu-Arg-Pro-Val-Leu-
              30   31                35
    Arg-**-Asn-Arg-Val-Glu-Tyr-*-Trp-*-Asn-
```

wherein the amino acids indicated by * and ** are not confirmed. However, the former is assumed to be Cys and the latter Ser.

(5) Substrate specificity:

The plasminogen activator hydrolyzed the synthetic substrate S-2251 in the presence of plasminogen, and more specifically hydrolyzed S-2288 than S-2444.

(6) Optimum pH: 10

(7) pH Stability:

The plasminogen activator is stable under a weak alkaline condition (pH: 8.0 -9.0).

(8) Thermal stability:

The plasminogen activator is stable at 45°C for 10 hours (at pH 9.0) and loses almost 100% activity within 30 minutes at 90°C and pH 9.0.

(9) Reaction velocity constant at 37°C and pH 8.4:

| Synthetic Substrate | Km (mol/l) | Kcat (sec$^{-1}$) |
|---|---|---|
| S-2288 | $4.3 \times 10^{-4}$ | 3.5 |
| S-2444 | $10.0 \times 10^{-4}$ | 1.9 |

Note: Km is the Michaelis constant and Kcat is the catalytic rate constant.

(10) Isoelectric point: 4.5 -7.2

As measured by the chromatofocusing method using Mono P column (Phamacia).

Example 2

$6 \times 10^5$ of KW cells were inoculated in a 75 cm² plastic flask, 30 ml of MEM containing a 10% fetal calf serum was added thereto, and then the cells were cultivated at 37°C for 7 days in an atmosphere of 5% $CO_2$ -95% air, to sufficiently proliferate the cells. The cells were washed with physiological saline, 10 ml of MEM free from serum was added thereto, and then the cells were cultivated for another 24 hours. Then, the TPA activity of the conditioned medium was determined to be 35 IU/ml.

Example 3

KW cells ($1 \times 10^7$) and 0.3 g of Cytodex I (Pharmacia) were introduced into a 100 ml spinner flask, 25 ml of MEM containing 10% of fetal calf serum was added thereto and then the cells were left to stand at 37°C in an atmosphere of 5% $CO_2$ -95% air. After a lapse of 3 hours, 75 ml of the same medium was added to the flask and the cultivation was commenced while stirring at 50 rpm. After sufficiently proliferating the cells on the beads, the serum medium was discarded and 80 ml of 199 medium containing 10 KIU/ml of aprotinin and free from serum was added to the flask and then the cultivation was conducted while stirring at 50 rpm, so as to produce TPA.

The TPA activity of the conditioned medium after the cultivation for 24 hours, was 25 IU/ml.

Example 4

$1 \times 10^7$ of KW cells were inoculated in each of 20 double trays of 1200 m² (Nunc), 180 ml of MEM containing a 10% fetal calf serum and 10 KIU/ml aprotinin was added thereto, and then the cells were cultivated at 37°C in an atmosphere of 5% $CO_2$ -95% air. After a lapse of 6 days, the culture medium was discarded, the cells were washed with physiological saline, and then the cultivation was conducted with 200ml of 199 medium containing 10KIU/ml aprotinin and 10mM HEPES buffer (N-2-hydroxyethylpiperazine-N'-2-ethansulfonic acid; pH 7.3) at 37°C in an atmosphere of 5% $CO_2$ -95% air, while whole medium replacement was made every day, so as to form TPA. After the cultivation for 18 days, 73.4 ℓ of the conditioned medium was recovered and the total TPA activity thereof was 600,000 IU. The resulting TPA was separated from the conditioned medium liquid and purified at 4°C according to the following procedures:

The conditioned medium was passed through a column of Zn-chelate-Sepharose (3.6 ℓ, the column size: I3 ˣ 27 cm) equilibrated with 20 mM Tris-HCl buffer (pH 7.5) including I M NaCl, 0.0I% Triton XI00, and I0 KIU/ml of aprotinin, at a flow rate of 3.6 ℓ/hr. After washing the column with the same buffer, a linear gradient from 0 to 50 mM imidazole in the same buffer was applied, so as to recover active fractions.

The active fractions obtained were adsorbed on concanavalin A-Sepharose column (200 ml, to column size: 3.95 ˣ I6.3 cm) equilibrated with I0 mM phosphate buffer (pH 7.5) containing I M NaCl, 0.0I% Triton XI00 and I0 KIU/ml of aprotinin). After washing the column with the same buffer, a linear gradient from the same buffer to I0mM phosphate buffer (pH 7.5) containing 2M KSCN, 0.0I% Triton ˣ I00, I0KIU/ml aprotinin, and 0.4M α-methyl-D-mannoside was applied. The eluate was concentrated by dialysis against solid polyethylene glycol 20,000. Then, the concentrate was subjected to gel filtration on Sephacryl S-200 - (Pharmacia; 580 ml, the column size: 2.9 ˣ 88 cm) equilibrated with I0 mM phosphate buffer containing I.6 MKSCN, 0.0I% Triton XI00 and I0 KIU/ml of aprotinin, so as to collect active fractions.

Then, these fractions were subjected to dialysis against I0 mM phosphate buffer (pH 7.5) containing I M NaCl, 0.0I% Triton XI00 and I0 KIU/ml of aprotinin, and adsorbed on lysine-Sepharose column (I25 ml, size: 3 ˣ I8 cm) equilibrated with the same buffer. After washing the column with the same buffer, TPA was eluted with the same buffer containing 50 mM lysine. The eluate was further subjected to gel filtration using the above-mentioned Sephacryl S-200 column. The preparation obtained was homogeneous electrophoretically.

These results were summarized in Table I.

Table I

| Procedures | Amount of Liquid (ml.) | Amount of Proteins* (mg) | Activity** (IU) | Specific Activity (IU/mg) | Yield (%) | Degree of Purification |
|---|---|---|---|---|---|---|
| Conditioned medium | 73,400 | 208,300 | 600,000 | 2.9 | 100 | 1.0 |
| Zn Chelate-Sepharose | 3,270 | 13,780 | 503,600 | 37 | 84 | 13 |
| Concanavalin A-Sepharose | 1,455 | 286 | 350,000 | 1,220 | 58 | 420 |
| Sephacryl S-200 | 74 | 66 | 266,400 | 4,000 | 44 | 1,380 |
| Lysine-Sepharose | 15 | 2.16 | 180,600 | 83,600 | 30 | 28,800 |
| Sephacryl S-200 | 37 | 1.584 | 150,000 | 94,700 | 25 | 32,700 |

* The amount of protein was determined according to Lowry method (see, J. Biol. Chem., 1951, 193, 265).

** The activity was measured according to the method described below based on UK as the Standard, and expressed as International Unit (IU) of UK.

0 227 102

(Technique for Measuring TPA Activity)

The measurement of TPA activity was carried out using a fibrin plate (22 ˣ 22 cm) prepared by adding I ml of thrombin solution (I0 U/ml) to 70 ml of a mixture containing both of 0.I5% plasminogen-containing fibrinogen solution (veronal buffer, pH 7.75) and 50 mM CaCl₂ solution each in the same amounts, with UK as the standard. TPA activity was calculated by multiplying I0 IU by the dilution ratio observed on the solution which provides the same fibrinolytic zone as that observed on the I0 IU/ml UK.

The present invention has been explained in detail with reference to the non-limitative and preferred examples. However, it is not intended to restrict the scope of this invention to those specific embodiments. On the contrary, it should be appreciated that the present invention includes various kinds of variations, alternatives, modifications and equivalents as may be included within the scope and spirit of this invention as defined by the appended claims.

**Claims**

I. A cell line (KW strain) established from human uterine muscle normal tissue and having the following properties:

I) Capable of producing a plasminogen activator which is immunologically different from urokinase;

2) Having estrogen receptors specific to the uterine cells;

3) Capable of subcultivation I20 times or more; and

4) Having a doubling time from I.4 to I.6 days.

2. KW strain IFO No. 50II3.

3. A method for producing a plasminogen activator comprises cultivating a cell line (KW strain) established from human uterine muscle normal tissue in a suitable culture medium containing serum to grow the cells, cultivating the resulting grown cells in a suitable culture medium free from serum, to obtain the plasminogen activator in the culture medium, and then separating and purifying the resultant plasminogen activator.

4. The method of claim 3, wherein the culture medium used in the growth culture process is a member selected from the group consisting of Eagle's MEM, Dulbecco's modified MEM, Hanks' I99 medium and Ham's medium which contain serum.

5. The method of claim 4, wherein said serum is fetal calf serum.

6. The method of claim 4, wherein said medium is Hanks' I99 medium.

7. The method of claim 6 wherein the culture medium used for producing the plasminogen activator is a member selected from the group consisting of Eagle's MEM, Dulbecco's MEM, Hanks' I99 medium, and Ham's medium which are free from serum.

8. The method of claim 7, wherein said medium is Hanks' I99 and MEM.

9. The method of claim 6, wherein the separation and purification of the plasminogen activator are carried out by using the procedure selected from the group consisting of adsorption chromatography, ion exchange chromatography, gel filtration, affinity chromatography techniques, fractional precipitation technique, membrane separation technique, electrophoresis and high pressure liquid chromatography.

I0. A plasminogen activator having the following properties:

(i) Specific activity: over 90,000 IU/mg-protein;

(ii) Molecular weight: 70,000 ± 2,000 (SDS-PAGE)

67,000 ± 5,000

(Gel filtration)

67,000 (HPLC):

(iii) Molecular structure: single-stranded structure;

(iv) N-Terminal amino acid sequence in the region I to 37 thereof

```
   1                 5                    10  11
   Ser-Tyr-Gln-Val-Ile-*-Arg-Asp-Glu-Lys-Thr-Gln-Met-Ile-
     15                   20  21                   25
   Tyr-Gln-Gln-His-Gln-**-Trp-Leu-Arg-Pro-Val-Leu-
               30  31                35
   Arg-**-Asn-Arg-Val-Glu-Tyr-*-Trp-*-Asn-
```

(The amino acids * and ** are assumed to be Cys and Ser, respectively.):

(v) Substrate specificity:

The plasminogen activator hydrolyzed the synthetic substrate S-2251 in the presence of plasminogen. Moreover, the plasminogen activator more specifically hydrolyzed the synthetic substrate S-2288 than the synthetic substrate S-2444;

(vi) Optimum pH: l0;

(vii) pH Stability:

The plasminogen activator is stable in a weak alkaline condition (pH 8.0 to 9.0);

(viii) Thermal stability;

The plasminogen activator is stable at 45°C for l0 hours (at pH 9.0) and loses almost l00% activity within 30 minutes at 90°C and pH 9.0;

(ix) Reaction velocity rate (at 37°C, pH 8.4):

```
Synthetic Substrate    Km (mol/l)      Kcat (sec⁻¹)

     S-2288           4.3 x 10⁻⁴          3.5

     S-2444          10.0 x 10⁻⁴          1.9
```

(wherein Km is the Michaelis constant and Kcat is the catalytic rate constant.

(x) Isoelectric Point: 4.5 to 7.2.